(19) Europäisches Patentamt
European Patent Office
Office européen des brevets

(11) Veröffentlichungsnummer: **0 071 009**
**B1**

(12) # EUROPÄISCHE PATENTSCHRIFT

(45) Veröffentlichungstag der Patentschrift: **04.02.87**

(51) Int. Cl.⁴: **C 07 D 249/08**, C 07 D 233/60, A 01 N 43/653, A 01 N 43/50

(21) Anmeldenummer: **82105171.1**

(22) Anmeldetag: **14.06.82**

(54) **Fungizide Alkinyl-azol-Derivate und deren Verwendung.**

(30) Priorität: **23.06.81 DE 3124580**

(43) Veröffentlichungstag der Anmeldung: **09.02.83 Patentblatt 83/6**

(45) Bekanntmachung des Hinweises auf die Patenterteilung: **04.02.87 Patentblatt 87/6**

(84) Benannte Vertragsstaaten: **BE CH DE FR GB IT LI NL**

(56) Entgegenhaltungen:
EP-A-0 019 153
EP-A-0 021 345
EP-A-0 025 516
EP-A-0 052 424

(73) Patentinhaber: **BAYER AG, Konzernverwaltung RP Patentabteilung, D-5090 Leverkusen 1 Bayerwerk (DE)**

(72) Erfinder: **Jäger, Gerhard, Dr., Gellertstrasse 18, D-5090 Leverkusen 1 (DE)**
Erfinder: **Büchel, Karl Heinz, Prof. Dr., Dabringhausener Strasse 42, D-5093 Burscheid (DE)**
Erfinder: **Krämer, Wolfgang, Dr., Am Eckbusch 39/45, D-5600 Wuppertal 1 (DE)**
Erfinder: **Frohberger, Paul- Ernst, Dr., Willi-Baumeister- Strasse 5, D-5090 Leverkusen 1 (DE)**
Erfinder: **Brandes, Wilhelm, Dr., Eichendorffstrasse 3, D-5653 Leichlingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 071 009 B1

LIBER, STOCKHOLM 1987

**Beschreibung**

Die Erfindung betrifft die Verwendung von teilweise bekannten Alkinyl-azol-Derivaten zur Bekämpfung von phytopathogenen Pilzen. Außerdem betrifft die Erfindung neue Alkinyl-azol-Derivate und Verfahren zu deren Herstellung.

Es ist bereits bekannt geworden, daß 1-Phenoxy-1-triazolyl-alkanol-Derivate gute fungizide Eigenschaften aufweisen (vgl. DE-A 2 324 010). Die Wirkung dieser Verbindungen ist jedoch, insbesondere bei niedrigen Aufwandmengen bzw. -konzentrationen, in einigen Indikationsbereichen nicht immer voll befriedigend.

Aus der EP-A 0 025 516 sind bestimmte Triazolylalkohole und deren Verwendung als Zwischenprodukte zur Herstellung von Triazolyl-ethern mit pflanzenwuchsregulierender Wirksamkeit bekannt. Es werden jedoch keine Triazolylalkohole beschrieben, die eine Halogenalkinyl-Gruppe enthalten, in welcher die Dreifachbindung endständig ist und auch endständig durch Halogen substituiert ist. Ferner werden von den Triazolylalkoholen keine biologischen Eigenschaften erwähnt.

In der EP-A 0 019 153 werden Carbinole offenbart, die als Zwischenprodukte zur Synthese von Verbindungen mit fungizider Wirksamkeit dienen. Es werden aber keine Stoffe aufgeführt, in denen das Carbinol-Kohlenstoffatom eine Alkinyl-Gruppe sowie eine Alkyl-Gruppe oder eine gegebenenfalls substituierte Phenyl-Gruppe trägt, wenn gleichzeitig das zum Triazolyl-Rest benachbarte Kohlenstoffatom durch andere Gruppen als einen gegebenenfalls halogenierten Phenoxy-Rest substituiert ist. Im übrigen werden von den Carbinolen keine biologischen Eigenschaften erwähnt.

Gemäß EP-A 0 021 345 lassen sich bestimmte Azol-Derivate, in denen das zum Azolyl-Rest benachbarte Kohlenstoffatom einen Hydroxy- oder Alkoxy-Rest trägt, als Zwischenprodukte einsetzen. Entsprechende Stoffe, in denen das in Bezug auf den Azolyl-Rest α-ständige Kohlenstoffatom andere Substituenten als Hydroxy oder Alkoxy aufweist, werden allerdings nicht beschrieben.

Schließlich sind aus der EP-A 0 052 424 2-Hydroxyethyl-azol-Derivate mit fungiziden und pflanzenwuchsregulierenden Eigenschaften bekannt. Es werden allerdings keine Stoffe offenbart, in denen das Kohlenstoffatom zwischen der Carbinol-Gruppe und dem Azolyl-Rest substituiert ist.

Es wurde nun gefunden, daß sich die teilweise bekannten Alkinyl- azol-Derivate der Formel (I)

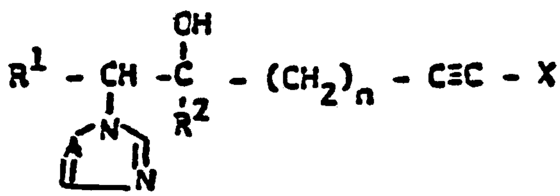

(I)

in welcher

$R^1$ für gegebenenfalls einfach oder mehrfach, gleich oder verschieden substituiertes Aryl oder Aryloxy mit jeweils 6 bis 10 Kohlenstoffatomen steht, wobei als Substituenten Halogen, Alkyl mit 1 bis 4 Kohlenstoffatomen, Alkoxy mit 1 bis 4 Kohlenstoffatomen, Nitro, Halogenalkyl mit 1 bis 2 Kohlenstoffatomen und 1 bis 5 gleichen oder verschiedenen Halogenatomen, sowie gegebenenfalls durch Halogen substituiertes Phenyl zu nennen sind,

$R^2$ für geradkettiges oder verzweigtes Alkyl mit 1 bis 4 Kohlenstoffatomen steht, ferner für durch Halogen substituiertes tert.-Butyl oder gegebenenfalls einfach oder mehrfach, gleich oder verschieden substituiertes Aryl mit 6 bis 10 Kohlenstoffatomen steht, wobei als Substituenten Halogen, Alkyl mit 1 bis 4 Kohlenstoffatomen, Alkoxy mit 1 bis 4 Kohlenstoffatomen, Nitro, Halogenalkyl mit 1 bis 2 Kohlenstoffatomen und 1 bis 5 gleichen oder verschiedenen Halogenatomen, sowie gegebenenfalls durch Halogen substituiertes Phenyl zu nennen sind,

X für Wasserstoff, Brom oder Jod steht,

A für Stickstoff oder die CH-Gruppe steht, und

n für die Zahlen 0 oder 1 steht,

sowie deren Säureadditions-Salze und Metallsalz-Komplexe zur Bekämpfung von phytopathogenen Pilzen verwenden lassen.

Die Verbindungen der Formel (I) besitzen zwei asymmetrische Kohlenstoffatome; sie können deshalb in zwei geometrischen Isomeren (threo- und erythro-Form) vorliegen, die in unterschiedlichen Mengenverhältnissen anfallen können.

Überraschenderweise zeigen die erfindungsgemäß zu verwendenden Alkinyl-azol-Derivate eine bessere Wirksamkeit gegen phytopathogene Pilze als die aus dem Stand der Technik bekannten 1-Phenoxy-1-triazolyl-alkanol-Derivate, welche wirkungsmäßig naheliegende Verbindungen sind. Die erfindungsgemäße Verwendung der weitgehend neuen Stoffe der Formel (I) stellt somit eine Bereicherung der Technik dar.

Die erfindungsgemäß zu verwendenden Alkinyl-azol-Derivate sind durch die Formel (I) allgemein definiert. Bevorzugt sind Verbindungen der Formel (Ia)

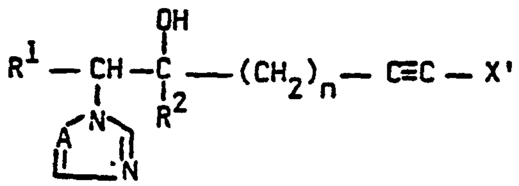

(I a)

in welcher

$R^1$ für gegebenenfalls einfach oder mehrfach, gleich oder verschieden substituiertes Aryl oder Aryloxy mit jeweils 6 bis 10 Kohlenstoffatomen steht, wobei als Substituenten genannt seien: Halogen, Alkyl mit 1 bis 4 Kohlenstoffatomen, Alkoxy mit 1 bis 4 Kohlenstoffatomen, Nitro, Halogenalkyl mit 1 bis 2 Kohlenstoffatomen und 1 bis 5 gleichen oder verschiedenen Halogenatomen, wie Fluor und Chlor, sowie gegebenenfalls durch Halogen substituiertes Phenyl;

$R^2$ für geradkettiges oder verzweigtes Alkyl mit 1 bis 4 Kohlenstoffatomen, durch Halogen substituiertes tert.-Butyl oder gegebenenfalls einfach oder mehrfach, gleich oder verschieden substituiertes Aryl mit 6 bis 10 Kohlenstoffatomen steht, wobei als Substituenten die bei $R^1$ bereits genannten Arylsubstituenten infrage kommen;

X' für Brom oder Jod steht, sowie auch für Wasserstoff steht, wenn nicht gleichzeitig $R^1$ für gegebenenfalls substituiertes Aryloxy, $R^2$ für Alkyl oder gegebenenfalls substituiertes Aryl und A für ein Stickstoffatom stehen; und

A und der Index n die in der Erfindungsdefinition angegebene Bedeutung haben.

Bevorzugt sind außerdem Verbindungen der Formel (Ib)

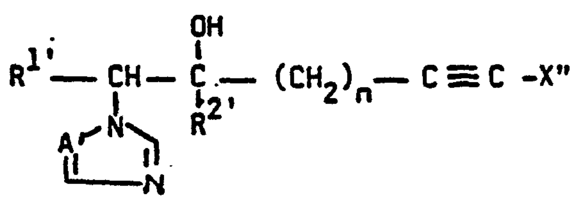

(I b)

in welcher

$R^{1'}$ für gegebenenfalls einfach oder mehrfach, gleich oder verschieden substituiertes Aryloxy mit 6 bis 10 Kohlenstoffatomen steht, wobei als Substituenten die bei $R^1$ bereits genannten Arylsubstituenten infrage kommen jedoch mit der Maßgabe, daß die Substitution nicht nur aus Chlorsubstituenten bestehen darf;

$R^{2'}$ für geradkettiges oder verzweigtes Alkyl mit 1 bis 4 Kohlenstoffatomen oder für gegebenenfalls einfach oder mehrfach, gleich oder verschieden substituiertes Aryl mit 6 bis 10 Kohlenstoffatomen steht, wobei als Substituenten die bei $R^1$ bereits genannten Arylsubstituenten infrage kommen;

X" für Wasserstoff steht;

A' für Stickstoff steht und

n für die Zahlen 0 oder 1 steht.

Bevorzugt sind weiterhin Verbindungen der Formel (Ic)

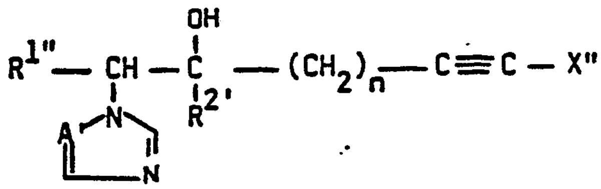

(I c)

in welcher

$R^{1''}$ für einfach oder mehrfach durch Chlor substituiertes Aryloxy mit 6 bis 10 Kohlenstoffatomen steht und

$R^{2'}$, X" A' und n die oben angegebene Bedeutung haben.

Bevorzugt sind ferner die Säureadditionssalze und Metallsalz-Komplexe der Verbindungen der Formel (I).

Besonders bevorzugt sind Verbindungen der Formel (Ia), in welcher

$R^1$ für gegebenenfalls einfach bis dreifach, gleich oder verschieden substituiertes Phenyl oder Phenoxy steht, wobei als Substituenten genannt seien: Fluor, Chlor, Brom, Methyl, Methoxy, Nitro, Trifluormethyl, Phenyl und Chlorphenyl;

$R^2$ für gegebenenfalls einfach oder zweifach durch Fluor, Chlor oder Brom substituiertes tert.-Butyl oder für gegebenenfalls einfach bis dreifach, gleich oder verschieden substituiertes Phenyl steht, wobei als Substituenten die bei $R^1$ bereits genannten Phenylsubstituenten infrage kommen;

X' für Brom oder Jod steht, sowie auch für Wasserstoff steht, wenn nicht gleichzeitig $R^1$ für gegebenenfalls substituiertes Phenoxy, $R^2$ für tert-Butyl oder gegebenenfalls substituiertes Phenyl und A für ein Stickstoffatom stehen; und

A und der Index n die in der Erfindungsdefinition angegebene Bedeutung haben.

Besonders bevorzugt sind außerdem Verbindungen der Formel (Ib) in welcher

$R^{1'}$ für gegebenenfalls einfach bis dreifach, gleich oder verschieden substituiertes Phenoxy steht, wobei als Substituenten die bei $R^1$ bereits genannten Phenylsubstituenten infrage kommen jedoch mit der Maßgabe, daß die Substitution nicht nur aus Chlorsubstituenten bestehen darf;

$R^{2'}$ für tert.-Butyl oder für gegebenenfalls einfach bis dreifach, gleich oder verschieden substituiertes Phenyl steht, wobei als Substituenten die bei $R^1$ bereits genannten Phenylsubstituenten infrage kommen;

X", A' und der Index n die oben angegebene Bedeutung haben.

Besonders bevorzugt sind weiterhin Verbindungen der Formel (Ic), in welcher

$R^{1''}$ für einfach oder zweifach durch Chlor substituiertes Phenoxy steht und

$R^{2'}$, X", A' und der Index n die oben angegebene Bedeutung haben.

Die erfindungsgemäß zu verwendenden Wirkstoffe der Formel (I) sind teilweise bekannt (zu verbindungen der Formel (Ic) vergleiche DE-A 2 918 801, zu verbindungen der Formel (Ib) vergleiche ebenfalls die in der DE-A 2 918 801 genannten Verbindungen ähnlicher Konstitution).

Die Verbindungen der Formel (Ia) sowie deren Säureadditions-Salze und Metallsalz-Komplexe sind neu. Sie können in allgemein üblicher und bekannter Art und Weise erhalten werden, indem man Azolyl-Ketone der Formel (II)

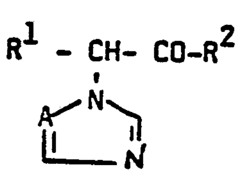

(II)

in welcher

A, $R^1$ und $R^2$ die oben angegebene Bedeutung haben,

a) für den Fall, daß n = 0 ist,

mit Mono-Alkalimetall-Salzen bzw. Mono-Grignard-Verbindungen des Acetylens in Gegenwart eines aprotischen organischen Lösungsmittels, wie z.B. Tetrahydrofuran, bei Temperaturen zwischen 0 und 50°C umsetzt und anschließend das Reaktionsgemisch hydrolisiert, oder

b) für den Fall, daß n = 1 ist,
mit Propargylhalogeniden in Gegenwart von aktiviertem Aluminium und in Gegenwart eines aprotischen organischen Lösungsmittels, wie z.B. Tetrahydrofuran, bei Temperaturen zwischen -70 und +30°C umsetzt, und gegebenenfalls die nach den Verfahren (a) und (b) erhaltenen Verbindungen der Formel (Id)

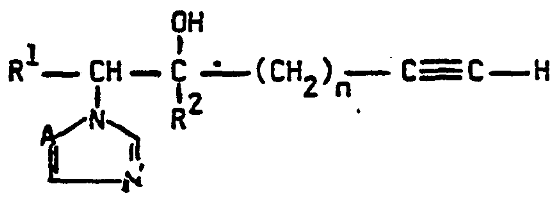

(I d)

in welcher
A, $R^1$, $R^2$ und der Index n die oben angegebene Bedeutung haben,
in üblicher Art und Weise mit Brom oder Jod in Gegenwart eines Verdünnungsmittels, wie beispielsweise Methanol, bei Temperaturen zwischen 0 und 50°C umsetzt (vergleiche auch die Herstellungsbeispiele) und gegebenenfalls noch an die so erhaltenen Verbindungen der Formel (Ia) eine Säure oder ein Metallsalz addiert.

Eine eventuelle Trennung der Diastereomerengemische kann in üblicher Weise erfolgen, wie z.B. durch fraktionierte Kristallisation oder durch chromatographische Trennmethoden.

Die Azolyl-Ketone der Formel (II) und deren Herstellung sind schon seit längerer Zeit bekannt (vergleiche z.B. DE-B 2 105 490, DE-B 2 201 063, DE-A 2 431 407, DE-A 2 632 603 und DE-A 2 632 602).

Zur Herstellung von physiologisch verträglichen Säureadditionssalzen der Verbindungen der Formel (I) kommen vorzugsweise folgende Säuren infrage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Milchsäure, sowie Sulfonsäuren, wie z.B. p-Toluolsulfonsäure und 1,5-Naphthalindisulfonsäure. Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen Verbindungen der Formel (I). Man kann die Metallsalz-Komplexe in bekannter Weise, z.B. durch Abfiltrieren, isolieren und gegebenenfalls durch Umkristallisieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z.B. gegen den Erreger des Gersten- bzw. Getreidemehltaus (Erysiphe graminis), von Sphaerotheca-Arten, wie z.B. gegen den Erreger des Gurkenmehltaus (Sphaerotheca fuliginea), sowie von Podosphaera-Arten, wie z.B. gegen den Erreger des Apfelmehltaus (Podosphaera leucotricha); außerdem auch zur Bekämpfung solcher Pilze, die Rostkrankheiten hervorrufen, so zur Bekämpfung von Puccinia-Arten, wie z.B. gegen den Erreger des Braunrostes am Weizen (Puccinia recondita).

In entsprechenden Aufwandmengen zeigen die erfindungsgemäßen Stoffe auch herbizide Eigenschaften.

Die Wirkstoffe können in die üblichen Formulierungen übergeführt werden wie Lösungen Emulsionen,

Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Tragerstoffe kommen in Frage z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

**Herstellungsbeispiele**

**Beispiel 1**

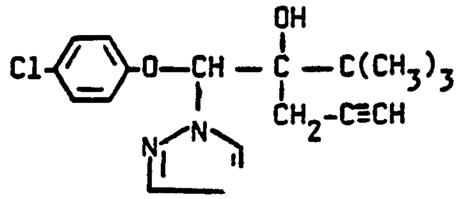

Man überschichtet 26 g (0,96 Mol) schuppenförmiges Aluminium mit 120 ml Tetrahydrofuran und gibt einige Körnchen Jod und eine Spatelspitze Quecksilber (II)-chlorid zu. Nach 12 Stunden läßt man unter kräftigem Rühren bei 60°C eine Lösung von 169 g (1,42 Mol) Propargyl bromid in 170 ml Tetrahydrofuran zutropfen. Anschließend wird auf -60°C gekühlt und im Verlaufe von 2 Stunden eine Lösung von 293,8 g (1 Mol) 1-(4-Chlorphenoxy)-3,3-dimethyl-1-(1H-1,2,4-triazol-1-yl)-2-butanon in 330 ml Tetrahydrofuran eingerührt. Das Reaktionsgemisch wird anschließend bei 0°C mit 340 ml einer gesättigten Ammoniumchlorid-Lösung versetzt. Das Lösungsmittel wird zum größten Teil unter vermindertem Druck abdestilliert und der Rückstand zweimal mit je 600 ml Essigester extrahiert. Nach dem Waschen mit Wasser wird die organische Phase über wasserfreiem Natriumsulfat getrocknet und anschließend unter vermindertem Druck eingeengt. Das ausgeschiedene, kristalline Produkt wird abfiltriert und mit Isopropanol und Diethylether gewaschen. Man erhält 131,1 g (39,3 % der Theorie) 4-((4-Chlorphenoxy)-(1-H-1,2,4-triazol-1-yl)-methyl)-5,5-dimethyl-1-hexin-4-ol vom Schmelzpunkt 124-125°C..

**Beispiel 2**

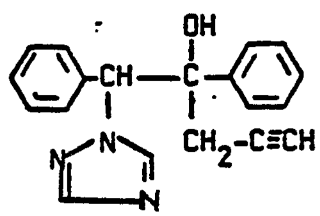

Analog Beispiel 1 erhält man aus 2,3 g (0,084 Mol) Aluminium, 14,7 g (0,124 Mol) Propargylbromid und 23 g (0,087 Mol) ω-Phenyl-ω-(1H-1,2,4-triazol-1-yl)-acetophenon in Tetrahydrofuran - 8,8 g (33,3 % der Theorie) 4,5-Diphenyl-5-(1H-1,2,4-triazol-1-yl)-1-pentin-4-ol als farblose Kristalle vom Schmelzpunkt 119-120°C.

**Beispiel 3**

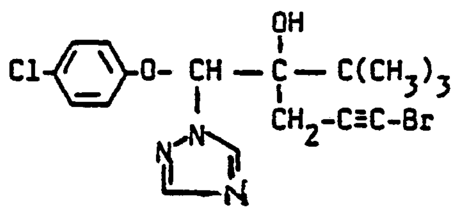

Zu einer Lösung von 7,7 g (0,048 Mol) Brom in 40 ml 10%iger Natronlauge werden bei 0°C 14 g (0,04 Mol) 4-((4-Chlorphenoxy)-(1H-1,2,4-triazol-1-yl))-methyl-5,5-dimethyl-1-hexin-4-ol (Beispiel 1), gelöst in 75 ml Dioxan, zugetropft. Nach fünf Stunden wird das Reaktionsgemisch in Eiswasser eingerührt. Das sich abscheidende Produkt wird abgesaugt, mit Wasser gewaschen und getrocknet. Man erhält 14,2 g (86,1 % der Theorie) 1-Brom-4-((4-Chlorphenoxy)-(1H-1,2,4-triazol-1-yl)-methyl)-5,5-dimethyl-1-hexin-4-ol vom Schmelzpunkt 156-160°C.

**Beispiel 4 und 5**

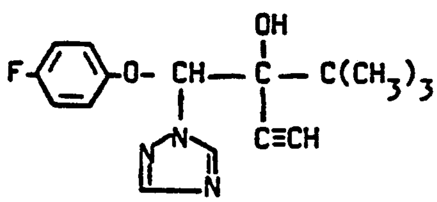

Beispiel 4: Diastereomerengemisch
Beispiel 5: Form A (eines der beiden möglichen geometrischen Isomeren)
In eine Suspension von 31,4 g (0,28 Mol) Kalium-tert-butylat in 300 ml Tetrahydrofuran wird 30 Minuten lang bei 15°C Acetylen unter Rühren eingeleitet. Anschließend wird unter weiterem Durchleiten von Acetylen eine Lösung von 55,5 g (0,2 Mol) 1-(4-Fluorphenoxy)-3,3-dimethyl-1-(1H-1,2,4-triazol-1-yl)-2-butanon in 150 ml Tetrahydrofuran in einer Stunde zugetropft. Nach weiteren zwei Stunden wird die Lösung durch Zugabe von 175 ml einer 23%igen wässrigen Ammoniumchlorid-Lösung auf einen pH-Wert von 8 eingestellt. Man extrahiert zweimal mit je 200 ml Essigester, wäscht mit Wasser und engt die Lösung unter vermindertem Druck ein. Der verbleibende feste Rückstand wird mit Petrolether verrieben. Man erhält so 27,2 g (44,8% der Theorie) 3-((4-Fluorphenoxy)-(1H-1,2,4-triazol-1-yl)-methyl)-4,4-dimethyl-1-pentin-3-ol vom Schmelzpunkt 155-161°C. Aus der Petroletherphase werden durch Kühlen und Anreiben noch 3,2 g (5,3% der Theorie) als eine reine diastereomere Form A vom Schmelzpunkt 134-135°C erhalten.

**Beispiel 6**

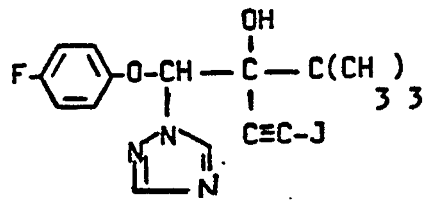

Zu einer Lösung von 14,7 g (0,049 Mol) 3-((4-Fluorphenoxy)-(1H-1,2,4-triazol-1-yl)-methyl)-4,4-dimethyl-1-pentin-3-ol (Beispiel 4) in 250 ml Methanol werden bei 20-25°C unter Rühren 12,5g (0,049 Mol) Jod portionsweise eingetragen und dabei gleichzeitig 20 ml konzentrierte Natronlauge zugetropft. Nach 12 Stunden wird die Lösung filtriert und das Filtrat in 500 ml Wasser eingerührt. Das ausgeschiedene, halbfeste Produkt wird in Essigester aufgenommen. Die Lösung wird mit Wasser gewaschen und nach dem Trocknen über wasserfreiem Natriumsulfat unter vermindertem Druck eingedampft. Der harzige Rückstand wird in wenig Diethylether gelöst und mit Petrolether versetzt. Das auskristallisierte Produkt wird abfiltriert und mit Petrolether gewaschen. Man erhält 15,1 g (71,9 % der Theorie) 1-Jod-3-((4-fluorphenoxy)-(1H-1,2,4-triazol-1-yl))-methyl-4,4-dimethyl-1-pentin-3-ol als farblose Kristalle vom Schmelzpunkt 140 bis 149°C.

In entpsrechender Weise werden die nachfolgenden Beispiele der allgemeinen Formel (I)

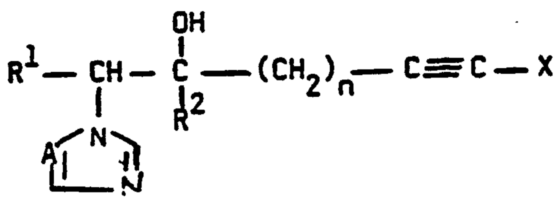

(I)

erhalten:

| Beispiel Nr. | $R^1$ | $R^2$ | A | n | X | Schmelzpunkt (°C) |
|---|---|---|---|---|---|---|
| 7 | 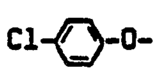 | $-C(CH_3)_3$ | N | 0 | H | 153-55 (A-Form)* |
| 8 | 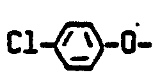 | $-C(CH_3)_3$ | N | 0 | H | 133-43 |
| 9 | 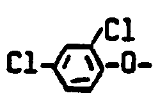 | $-C(CH_3)_3$ | N | 0 | H | 163-66 (A-Form)* |
| 1o | 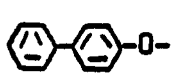 | $-C(CH_3)_3$ | N | 0 | H | 187-89 |
| 11 | 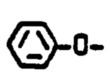 | $-C(CH_3)_3$ | N | 0 | H | 191-93 |
| 12 | 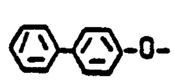 | $-C(CH_3)_3$ | N | 0 | J | 148-53 |

| Beispiel Nr. | $R^1$ | $R^2$ | A | n | X | Schmelzpunkt (°C) |
|---|---|---|---|---|---|---|
| 13 | 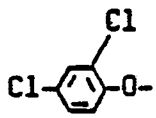 | $-C(CH_3)_3$ | N | 1 | H | Harz |
| 14 | 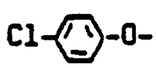 | 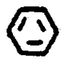 | N | 1 | H | 157-58 |
| 15 | 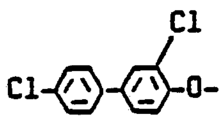 | $-C(CH_3)_3$ | N | 1 | H | Harz |
| 16 | 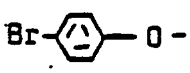 | $-C(CH_3)_2CH_2Br$ | N | 1 | H | 127 |
| 17 | 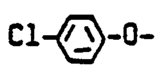 | $-C(CH_3)_3$ | N | 1 | J | 2o1 |
| 18 | 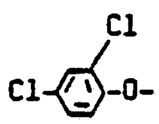 | $-C(CH_3)_3$ | N | 1 | J | 176-77 |
| 19 | 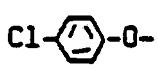 | 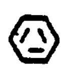 | N | 1 | J | 171-72 |
| 2o | 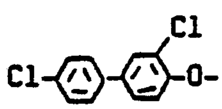 | $-C(CH_3)_3$ | N | 1 | J | 156-58 |
| 21 | 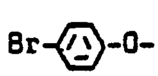 | $-C(CH_3)_2CH_2Br$ | N | 1 | J | 194-95 |
| 22 | 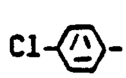 | 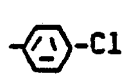 | N | 1 | J | 157-58 |

| Beispiel Nr. | $R^1$ | $R^2$ | A | n | X | Schmelzpunkt (°C) |
|---|---|---|---|---|---|---|
| 23 | 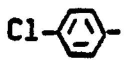 | 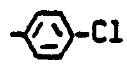 | N | 1 | H | 176-77 |
| 24 | 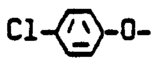 | $-C(CH_3)_3$ | CH | O | H | 1oo-2o |
| 25 | 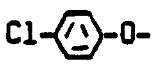 | $-C(CH_3)_3$ | CH | O | H | 143-44 (B-Form)* |
| 26 | 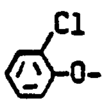 | $-C(CH_3)_3$ | CH | O | H | Harz |
| 27 | 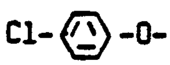 | $-C(CH_3)_3$ | CH | O | J | 8o-1o5 |
| 28 | 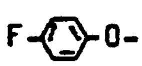 | $-C(CH_3)_3$ | N | O | H | 160-68 (B-Form)* |

*A- und B-Form: die beiden möglichen geometrischen Isomeren

**Verwendungsbeispiele**

In den nachfolgenden Beispielen werden die nachstehend angegebenen Verbindungen als Vergleichssubstanzen eingesetzt:

(A)

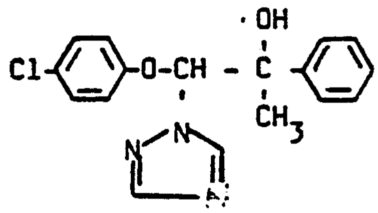

(B)

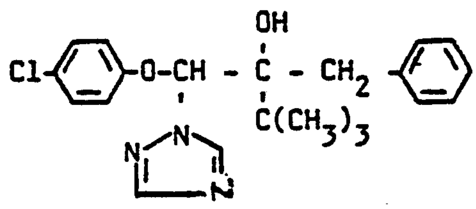

(C)

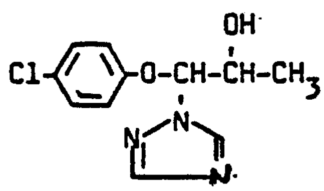

(D)

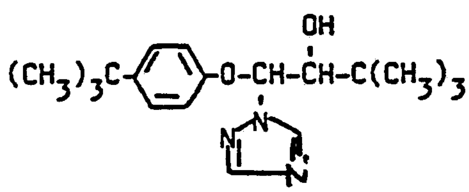

**Beispiel A**

Sphaerotheca-Test (Gurke) / protektiv /
Lösungsmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca.75% im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 8,7 und 1.

**Tabelle A**

Sphaerotheca-Test (Gurke) / protektiv /

| Wirkstoff | | Befall in % bei einer Wirkstoffkonzentration von 0,0001 % |
|---|---|---|
| 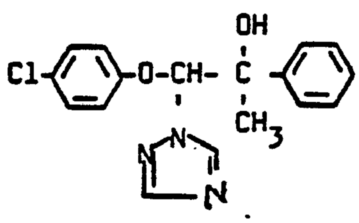 (bekannt) | (A) | 54 |
| 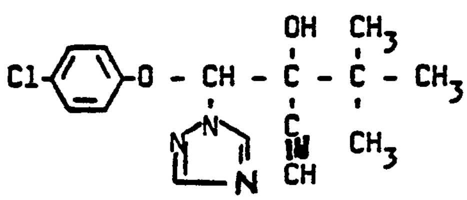 | (8) | 15 |
| 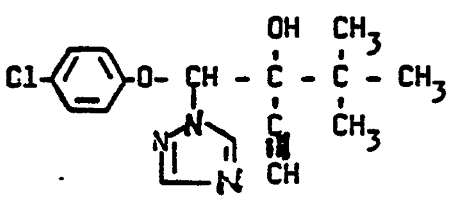 | (7) | 29 |
| 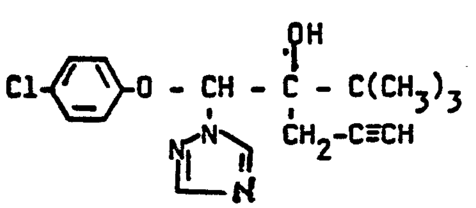 | (1) | 34 |

**Beispiel B**

Podosphaera-Test (Apfel) / protektiv /
Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23°C und einer relativen Luftfeuchtigkeit von ca. 70 % aufgestellt.

9 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 5, 8, 7, 1 und 16.

**Tabelle B**

Podosphaera-Test (Apfel) / protektiv /

| Wirkstoff | | Befall in % bei einer Wirkstoffkonzentration von 0,001 % |
|---|---|---|
| 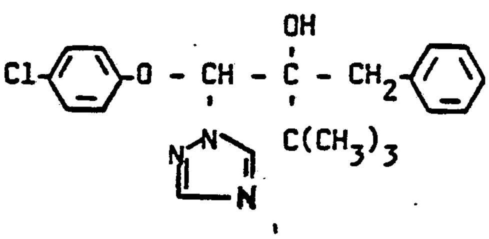 (bekannt) | (8) | 14 |
| 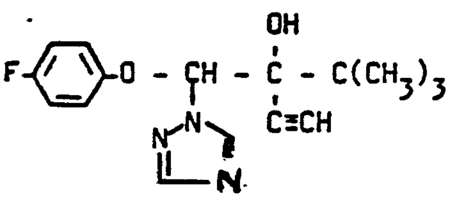 (A-Form) | (5) | 0 |
| 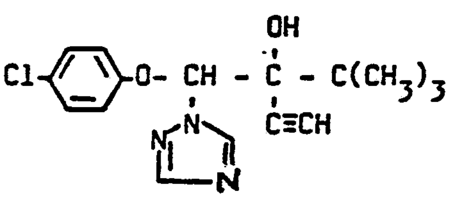 | (8) | 1 |
| 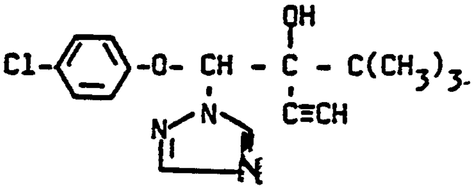 (A-Form) | (7) | 6 |

**Tabelle B**

Podosphaera-Test (Apfel) / protektiv /

| Wirkstoff | | Befall in % bei einer Wirkstoffkonzentration von 0,001 % |
|---|---|---|
| 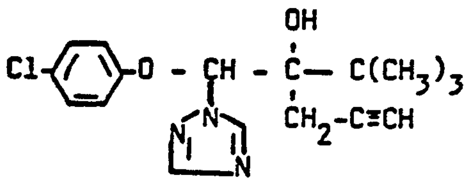 | (1) | 0 |
| 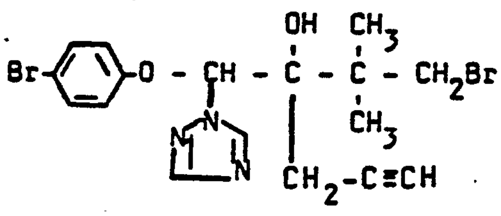 | (16) | 4 |

**Beispiel C**

Erysiphe-Test (Gerste) / protektiv /
Lösungsmittel: 100 Gewichtsteile Dimethylformamid
Emulgator: 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 4, 5, 8, 7, 10, 1, 13, 14, 16 und 3.

**Tabelle C**

Erysiphe-Test (Gerste) / protektiv /

| Wirkstoff | | Wirkstoffkonzentration in der Spritzbrühe in Gew.-% | Krankheitsbefall in % der unbehandelten Kontrolle |
|---|---|---|---|
| 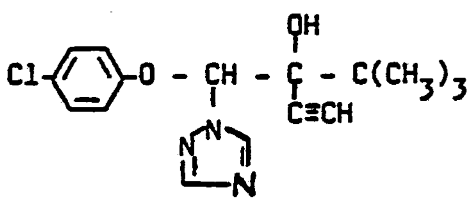 (A-Form) | (7) | o,oo25 | 0,0 |
| 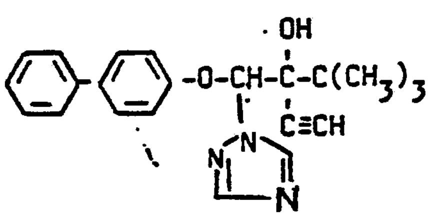 | (1o) | o,oo25 | 16,3 |
| 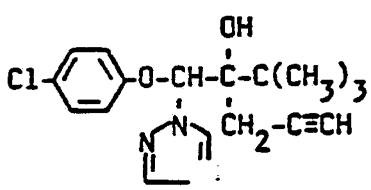 | (1) | o,oo25 | 0,0 |
| 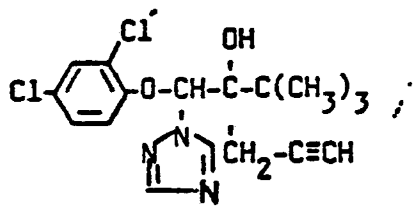 | (13) | o,oo25 | 0,0 |

**Tabelle C**

Erysiphe-Test (Gerste) / protektiv /

| Wirkstoff | Wirkstoffkonzentration in der Spritzbrühe in Gew.-% | Krankheitsbefall in % der unbehandelten Kontrolle |
|---|---|---|
| 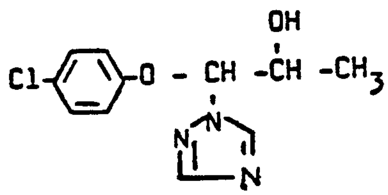 (C) (bekannt) | o,oo25 | loo |
| 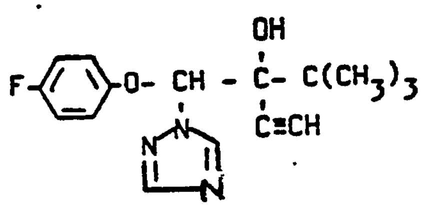 (4) | o,oo25 | 15,o |
| 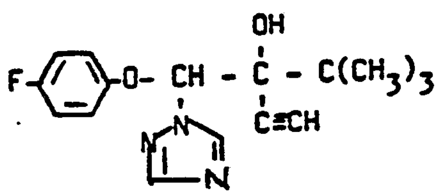 (5) (A-Form) | o,oo25 | 12,5 |
| 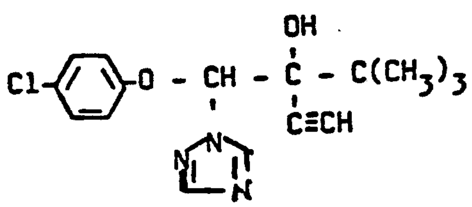 (8) Diastereomerengemisch | o,oo25 | 0,0 |

**Tabelle C**

Erysiphe-Test (Gerste) / protektiv /

| Wirkstoff | | Wirkstoffkonzentration in der Spritzbrühe in Gew.-% | Krankheitsbefall in % der unbehandelten Kontrolle |
|---|---|---|---|
| 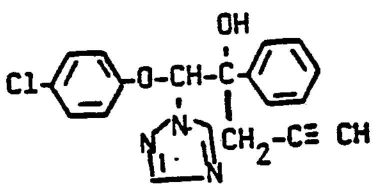 | (14) | o,oo25 | 12,5 |
| 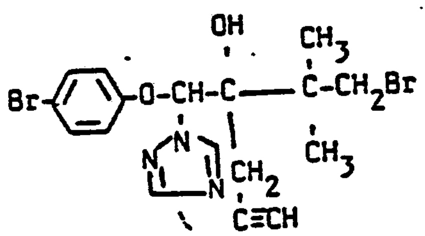 | (16) | o,oo25 | 8,8 |
| 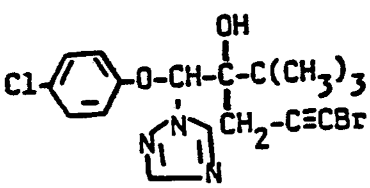 | (3) | o,oo25 | 8,8 |

**Beispiel D**

Erysiphe-Test (Gerste) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 x 12 Korn 2 cm tief in eine Standarderde. 7 Tage nach der Aussaat, wenn die jungen Pflanzen ihr erstes Blatt entfaltet haben, werden sie mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 4, 5, 8, 7, 1 und 14.

**Tabelle D**

Erysiphe-Test (Gerste) / Saatgutbehandlung

| Wirkstoff | | Wirkstoffaufwandmenge in mg/kg Saatgut | Krankheitsbefall in % der unbehandelten Kontrolle |
|---|---|---|---|
| 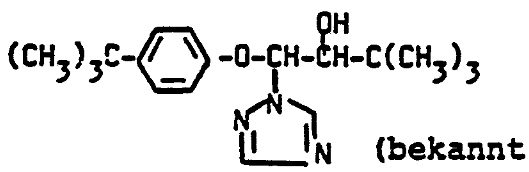 (bekannt) | (D) | 2500 | 100 |
| 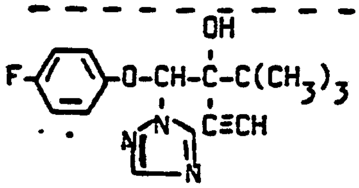 | (4) | 1000 | 0,0 |
| 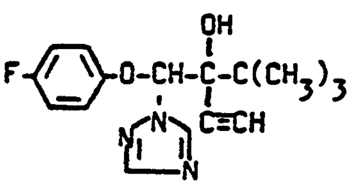 (A-Form) | (5) | 1000 | 0,0 |
| 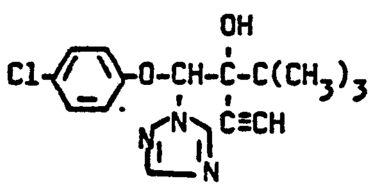 | (8) | 2500 | 0,0 |
| 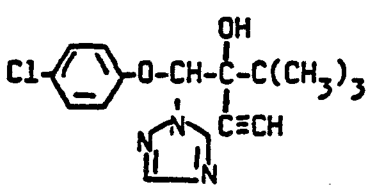 (A-Form) | (7) | 2500 | 0,0 |

**Tabelle D**

Erysiphe-Test (Gerste) / Saatgutbehandlung

| Wirkstoff | | Wirkstoff-aufwand-menge in mg/kg Saatgut | Krankheits-befall in % der unbehan-delten Kontrolle |
|---|---|---|---|
| 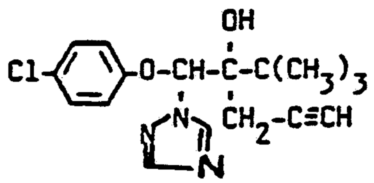 | (1) | 1000 | 0,0 |
| 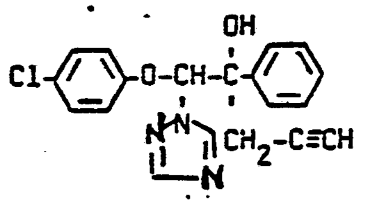 | (14) | 1000 | 16,3 |

**Beispiel E**

Puccinia-Test (Weizen) / protektiv /

Lösungsmittel: 100 Gewichtsteile Dimethylformamid

Emulgator: 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksankeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 8, 1, 13 und 3.

**Tabelle E**

Puccinia-Test (Weizen) / protektiv /

| Wirkstoff | | Wirkstoffkonzentration in der Spritzbrühe in Gew.-% | Krankheitsbefall in % der unbehandelten Kontrolle |
|---|---|---|---|
| 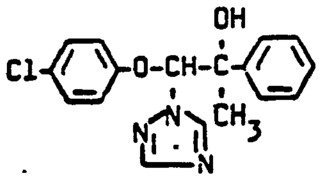 (bekannt) | (A) | o, o25 | loo |
| 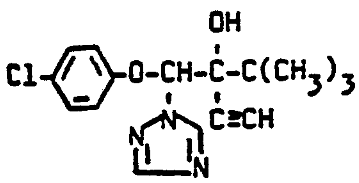 | (8) | o,o25 | 12,5 |
| 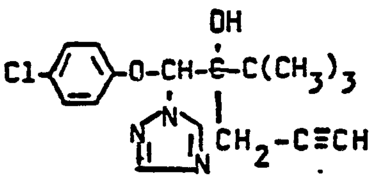 | (1) | o,o25 | 8,8 |
| 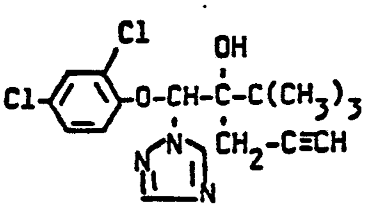 | (13) | o,o25 | 12,5 |
| 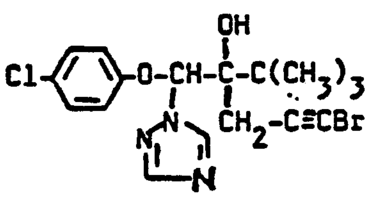 | (3) | o,o25 | 0,0 |

## Patentansprüche

1. Verwendung von Alkinyl-azol-Derivaten der Formel

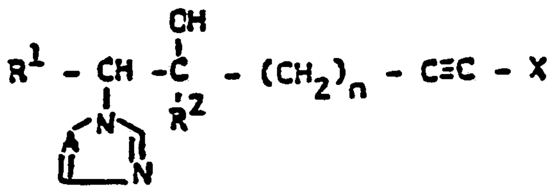

(I)

in welcher

$R^1$ für gegebenenfalls einfach oder mehrfach, gleich oder verschieden substituiertes Aryl oder Aryloxy mit jeweils 6 bis 10 Kohlenstoffatomen steht, wobei als Substituenten Halogen, Alkyl mit 1 bis 4 Kohlenstoffatomen, Alkoxy mit 1 bis 4 Kohlenstoffatomen, Nitro, Halogenalkyl mit 1 bis 2 Kohlenstoffatomen und 1 bis 5 gleichen oder verschiedenen Halogenatomen, sowie gegebenenfalls durch Halogen substituiertes Phenyl zu nennen sind,

$R^2$ für geradkettiges oder verzweigtes Alkyl mit 1 bis 4 Kohlenstoffatomen steht, ferner für durch Halogen substituiertes tert.-Butyl oder gegebenenfalls einfach oder mehrfach, gleich oder verschieden substituiertes Aryl mit 6 bis 10 Kohlenstoffatomen steht, wobei als Substituenten Halogen, Alkyl mit 1 bis 4 Kohlenstoffatomen, Alkoxy mit 1 bis 4 Kohlenstoffatomen, Nitro, Halogenalkyl mit 1 bis 2 Kohlenstoffatomen und 1 bis 5 gleichen oder verschiedenen Halogenatomen, sowie gegebenenfalls durch Halogen substituiertes Phenyl zu nennen sind,

X für Wasserstoff, Brom oder Jod steht,

A für Stickstoff oder die CH-Gruppe steht, und

n für die Zahlen 0 oder 1 steht,

sowie von deren Säureadditionssalzen und Metallsalzkomplexen zur Bekämpfung von phytopathogenen Pilzen.

2. Alkinyl-azol-Derivate der allgemeinen Formel (Ia)

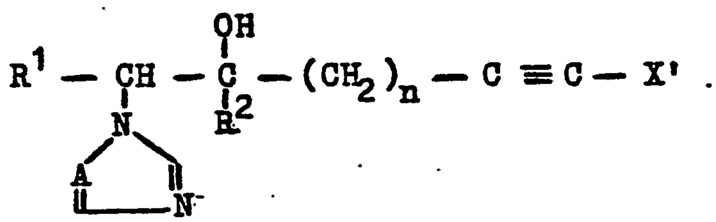

(Ia)

in welcher

$R^1$ für gegebenenfalls einfach oder mehrfach, gleich oder verschieden substituiertes Aryl oder Aryloxy mit jeweils 6 bis 10 Kohlenstoffatomen steht, wobei als Substituenten Halogen, Alkyl mit 1 bis 4 Kohlenstoffatomen, Alkoxy mit 1 bis 4 Kohlenstoffatomen, Nitro, Halogenalkyl mit 1 bis 2 Kohlenstoffatomen und 1 bis 5 gleichen oder verschiedenen Halogenatomen, sowie gegebenenfalls durch Halogen substituiertes Phenyl zu nennen sind,

$R^2$ für geradkettiges oder verzweigtes Alkyl mit 1 bis 4 Kohlenstoffatomen steht, ferner für durch Halogen substituiertes tert.-Butyl oder für gegebenenfalls einfach oder mehrfach, gleich oder verschieden substituiertes Aryl mit 6 bis 10 Kohlenstoffatomen steht, wobei als Substituenten Halogen, Alkyl mit 1 bis 4 Kohlenstoffatomen, Alkoxy mit 1 bis 4 Kohlenstoffatomen, Nitro, Halogenalkyl mit 1 bis 2 Kohlenstoffatomen und 1 bis 5 gleichen oder verschiedenen Halogenatomen, sowie gegebenenfalls durch Halogen substituiertes Phenyl zu nennen sind,

X' für Brom oder Jod steht, sowie auch für Wasserstoff steht, wenn nicht gleichzeitig $R^1$ für gegebenenfalls substituiertes Aryloxy, $R^2$ für Alkyl oder gegebenfalls substituiertes Aryl und A für ein Stickstoffatom stehen,

A für Stickstoff oder die CH-Gruppe steht, und

n für die Zahlen 0 oder 1 steht,

sowie deren Säureadditionssalze und Metallsalzkomplexe.

3. Alkinyl-azol-Derivate der allgemeinen Formel (Ia) in Anspruch 2, worin

$R^1$ für gegebenenfalls einfach bis dreifach, gleich oder verschieden substituiertes Phenyl oder Phenoxy steht, wobei als Substituenten genannt seien: Fluor, Chlor, Brom, Methyl, Methoxy, Nitro, Trifluormethyl, Phenyl und Chlorphenyl;

$R^2$ für gegebenenfalls einfach oder zweifach durch Fluor, Chlor oder Brom substituiertes tert.-Butyl oder für gegebenenfalls einfach bis dreifach, gleich oder verschieden substituiertes Phenyl steht, wobei als Substituenten die bei $R^1$ bereits genannten Phenylsubstituenten infrage kommen;

X' für Brom und Jod steht, sowie auch für Wasserstoff steht, wenn nicht gleichzeitig $R^1$ für gegebenenfalls substituiertes Phenoxy, $R^2$ für tert.-Butyl oder gegebenenfalls substituiertes Phenyl und A für ein Stickstoffatom stehen; und

A und der Index n die in Anspruch 2 angegebene Bedeutung besitzen.

4. Verfahren zur Herstellung von Alkinyl-azol-Derivaten der allgemeinen Formel (Ia)

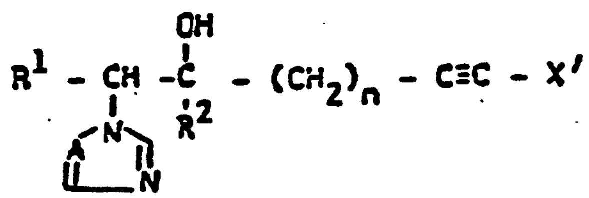

(Ia)

in welcher

$R^1$ für gegebenenfalls einfach oder mehrfach, gleich oder verschieden substituiertes Aryl oder Aryloxy mit jeweils 6 bis 10 Kohlenstoffatomen steht, wobei als Substituenten Halogen, Alkyl mit 1 bis 4 Kohlenstoffatomen, Alkoxy mit 1 bis 4 Kohlenstoffatomen, Nitro, Halogenalkyl mit 1 bis 2 Kohlenstoffatomen und 1 bis 5 gleichen oder verschiedenen Halogenatomen, sowie gegebenenfalls durch Halogen substituiertes Phenyl zu nennen sind,

$R^2$ für geradkettiges oder verzweigtes Alkyl mit 1 bis 4 Kohlenstoffatomen steht, ferner für durch Halogen substituiertes tert.-Butyl oder gegebenenfalls einfach oder mehrfach, gleich oder verschieden substituiertes Aryl mit 6 bis 10 Kohlenstoffatomen steht, wobei als Substituenten Halogen, Alkyl mit 1 bis 4 Kohlenstoffatomen, Alkoxy mit 1 bis 4 Kohlenstoffatomen, Nitro, Halogenalkyl mit 1 bis 2 Kohlenstoffatomen und 1 bis 5 gleichen oder verschiedenen Halogenatomen, sowie gegebenenfalls durch Halogen substituiertes Phenyl zu nennen sind,

X' für Brom oder Jod steht, sowie auch für Wasserstoff steht, wenn nicht gleichzeitig $R^1$ für gegebenenfalls substituiertes Aryloxy, $R^2$ für Alkyl oder gegebenenfalls substituiertes Aryl und A für ein Stickstoffatom stehen,

A für Stickstoff oder die CH-Gruppe steht, und

n für die Zahlen 0 oder 1 steht,

und deren Säureadditionssalze und Metallsalzkomplexe, dadurch gekennzeichnet, daß man Azolyl-Ketone der Formel (II)

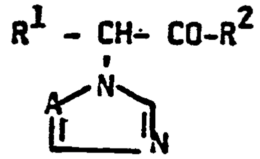 (II)

in welcher
A, $R^1$ und $R^2$ die oben angegebene Bedeutung haben,
a) für den Fall, daß n = 0 ist,
mit Mono-Alkalimetall-Salzen bzw. Mono-Grignard-Verbindungen des Acetylens in Gegenwart eines aprotischen organischen Lösungsmittels, wie z.B. Tetrahydrofuran, bei Temperaturen zwischen 0 und 50°C umsetzt und anschlißend das Reaktionsgemisch hydrolisiert, oder
b) für den Fall, daß n = 1 ist,
mit Propargylhalogeniden in Gegenwart von aktiviertem Aluminium und in Gegenwart eines aprotischen organischen Lösungsmittels, wie z.B. Tetrahydrofuran, bei Temperaturen zwischen -70 und +30°C umsetzt, und gegebenenfalls die nach den Verfahren (a) und (b) erhaltenen Verbindungen der Formel (Id)

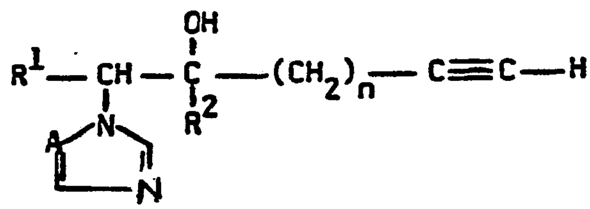 (Id)

in welcher
A, $R^1$, $R^2$ und der Index n die oben angegebene Bedeutung haben,
in üblicher Weise mit Brom oder Jod in Gegenwart eines Verdünnungsmittels, bei Temperaturen zwischen 0 und 50°C umsetzt, und gegebenenfalls noch an die so erhaltenen Verbindungen der Formel (Ia) eine Säure oder ein Metallsalz addiert.

**Claims**

1. Use of alkinyl-azole derivatives of the formula (I)

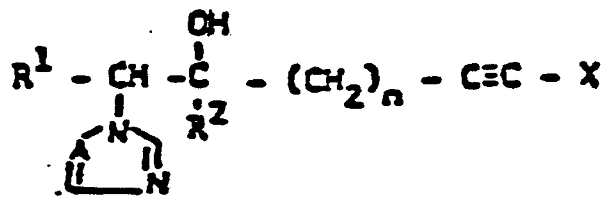

(I)

in which

$R^1$ represents aryl or aryloxy which in each case have 6 to 10 carbon atoms and are optionally mono- or polysubstituted by identical or different substituents, the substituents to be mentioned being halogen, alkyl with 1 to 4 carbon atoms, alkoxy with 1 to 4 carbon atoms, nitro, halogenoalkyl with 1 to 2 carbon atoms and 1 to 5 identical or different halogen atoms, and phenyl which is optionally substituted by halogen,

$R^2$ represents straight-chain or branched alkyl with 1 to 4 carbon atoms, and also represents halogensubstituted tert.-butyl or aryl which has 6 to 10 atoms and is optionally mono- or polysubstituted by identical or different substituents, the substituents to be mentioned being halogen, alkyl with 1 to 4 carbon atoms, alkoxy with 1 to 4 carbon atoms, nitro, halogenoalkyl with 1 to 2 carbon atoms and 1 to 5 identical or different halogen atoms, and phenyl which is optionally substituted by halogen,

X represents hydrogen, bromine or iodine,

A represents nitrogen or the CH group, and

n represents the numbers 0 or 1,

and their acid addition salts and metal salt complexes for combating phytopathogenic fungi.

2. Alkinyl-azole derivatives of the general formula (Ia)

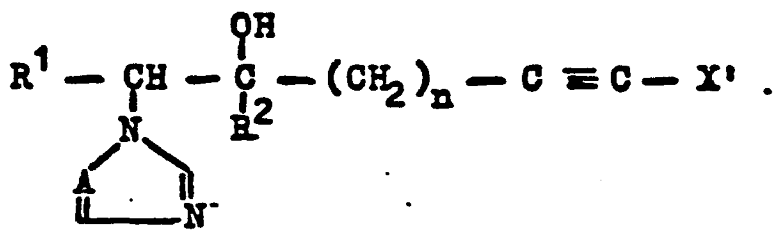

(Ia)

in which

$R^1$ represents aryl or aryloxy which in each case have 6 to 10 carbon atoms and are optionally mono- or polysubstituted by identical or different substitutents, the substituents to be mentioned being halogen, alkyl with 1 to 4 carbon atoms, alkoxy with 1 to 4 carbon atoms, nitro, halogenoalkyl with 1 to 2 carbon atoms and 1 to 5 identical or different halogen atoms, and phenyl which is optionally substituted by halogen,

$R^2$ represents straight-chain or branched alkyl with 1 to 4 carbon atoms, and also represents halogensubstituted tert.-butyl or aryl which has 6 to 10 carbon atoms and is optionally mono- or polysubstituted by identical or different substituents, the substituents to be mentioned being halogen, alkyl with 1 to 4 carbon atoms, alkoxy with 1 to 4 carbon atoms, nitro, halogenoalkyl with 1 to 2 carbon atoms and 1 to 5 identical or different halogen atoms, and phenyl which is optionally substituted by halogen,

X' represents bromine or iodine, and also represents hydrogen when $R^1$ does not simultaneously represent optionally substituted aryloxy, $R^2$ does not simultaneously represent alkyl or optionally substituted aryl and A does not simultaneously represent a nitrogen atom,

A represents nitrogen or the CH group and

n represents the numbers 0 or 1,

and their acid addition salts and metal salt complexes.

3. Alkinyl-azole derivatives of the general formula (Ia) in Claim 2, wherein

$R^1$ represents phenoxy or phenyl which is optionally mono- to trisubstituted by identical or different

substituents, the substituents which may be mentioned being: fluorine, chlorine, bromine, methyl, methoxy, nitro, trifluoromethyl, phenyl and chlorophenyl,

$R^2$ represents tert.-butyl which is optionally mono- or disubstituted by fluorine, chlorine or bromine, or phenyl which is optionally mono- to trisubstituted by identical or different substituents, possible substituents being the phenyl substituents already mentioned under $R^1$;

X' represents bromine and iodine, and also represents hydrogen when $R^1$ does not simultaneously represent optionally substituted phenoxy, $R^2$ does not simultaneously represent tert.-butyl or optionally substituted phenyl and A does not simultaneously represent a nitrogen atom; and

A and the index n have the meaning given in Claim 2.

4. Process for the preparation of alkinyl-azole derivatives of the general formula (Ia)

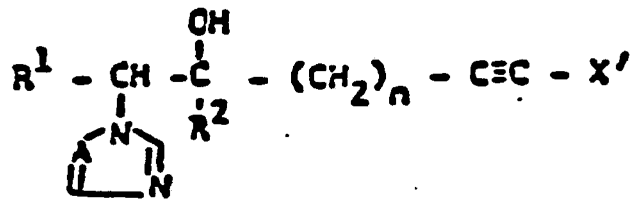

(Ia)

in which

$R^1$ represents aryl or aryloxy which in each case have 6 to 10 carbon atoms and are optionally mono- or polysubstituted by identical or different substituents, the substituents to be mentioned being halogen, alkyl with 1 to 4 carbon atoms, alkoxy with 1 to 4 carbon atoms, nitro, halogenoalkyl with 1 to 2 carbon atoms and 1 to 5 identical or different halogen atoms, and phenyl which is optionally substituted by halogen,

$R^2$ represents straight-chain or branched alkyl with 1 to 4 carbon atoms, and also represents halogensubstituted tert.-butyl or aryl which has 6 to 10 carbon atoms and is optionally mono- or polysubstituted by identical or different substituents, the substituents to be mentioned being halogen, alkyl with 1 to 4 carbon atoms, alkoxy with 1 to 4 carbon atoms, nitro, halogenoalkyl with 1 to 2 carbon atoms and 1 to 5 identical or different halogen atoms, and phenyl which is optionally substituted by halogen,

X' represents bromine or iodine, and also represents hydrogen when $R^1$ does not simultaneously represent optionally substituted aryloxy, $R^2$ does not simultaneously represent alkyl or optionally substituted aryl and A does not simultaneously represent a nitrogen atom,

A represents nitrogen or the CH group and

n represents the numbers 0 or 1,

and their acid addition salts and metal salt complexes, characterised in that azolyl ketones of the formula (II)

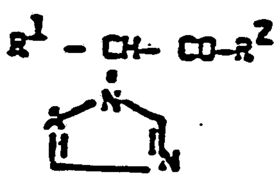

(II)

in which

A, $R^1$ and $R^2$ have the abovementioned meaning,

a) in the case where n is 0,

are reacted with mono-alkali metal salts or mono-Grignard compounds of acetylene in the presence of an aprotic organic solvent, such as for example tetrahydrofuran at temperatures between 0 and 50°C and the reaction mixture is then hydrolysed, or

b) in the case where n is 1,

are reacted with propargyl halides in the presence of activated aluminium and in the presence of an aprotic organic solvent, such as for example tetrahydrofuran, at temperatures between -70 and +30°C, and, if appropriate, the compounds obtained by the processes (a) and (b), of the formula (Id)

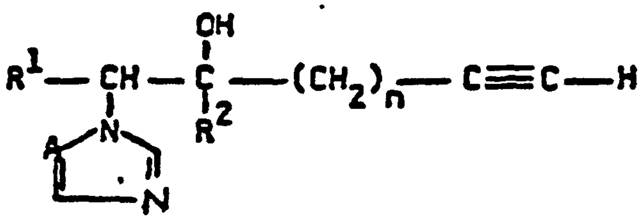

(Id)

in which

A, $R^1$, $R^2$ and the index n have the abovementioned meaning,

are reacted, in a customary manner, with bromine or iodine in the presence of a diluent, at temperatures between 0 and 50°C and, if desired, an acid or a metal salt is then added on to the compounds of the formula (Ia) thus obtained.

**Revendications**

1. Utilisation de dérivés alcinyl-azoliques de formule (I)

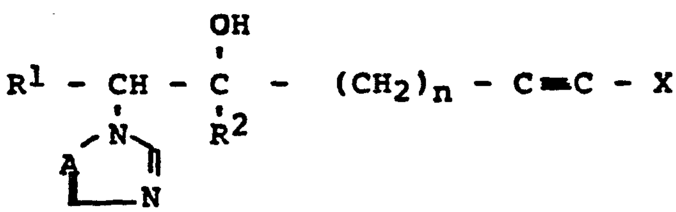

(I)

dans laquelle

$R^1$ représente un aryle ou aryloxy ayant chacun 6 à 10 atomes de carbone et eventuellement substitués une ou plusieurs fois, de manière identique ou différente, en citant comme substituants halogene, alcoyle ayant 1 à 4 atomes de carbone, alcoxy ayant 1 à 4 atomes de carbone, nitro, halogénoalcoyle ayant 1 à 2 atomes de carbone et 1 à 5 atomes d'halogène identiques ou différents, de même qu'un phényle éventuellement substitué par de l'halogène,

$R^2$ représente un alcoyle à chaîne droite ou ramifiée ayant 1 à 4 atomes de carbone, en outre un t-butyle substitué par de l'halogène ou un aryle éventuellement substitué une ou plusieurs fois, de manière identique ou différente et ayant 6 à 10 atomes de carbone, en citant comme substituants halogène, alcoyle ayant 1 à 4 atomes de carbone, alcoxy ayant 1 à 4 atomes de carbone, nitro, halogénoalcoyle ayant 1 à 2 atomes de carbone et 1 à 5 atomes d'halogène identiques ou différents, de même que phényle éventuellement substitué par de l'halogène,

X représente de l'hydrogène, du brome ou de l'iode,

A de l'azote ou le groupe CH et

n les nombres 0 ou 1,

de même que de leurs sels d'addition d'acides et leurs complexes avec des sels métalliques, pour combattre les champignons phytopathogenes.

2. Dérivés alcinyl-azoliques de formule generale (Ia):

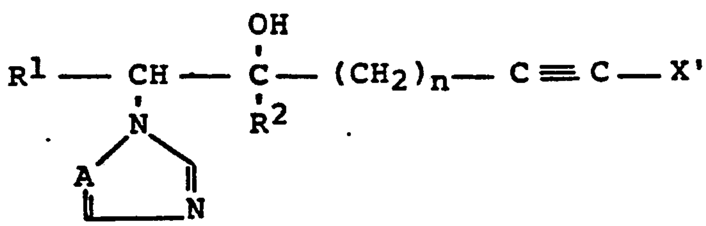 (Ia)

dans laquelle

$R^1$ représente un aryle ou aryloxy ayant chacun 6 à 10 atomes de carbone et eventuellement substitués une ou plusieurs fois, de manière identique ou différente, en citant comme substituants halogène, alcoyle ayant 1 à 4 atomes de carbone, alcoxy ayant 1 à 4 atomes de carbone, nitro, halogénoalcoyle ayant 1 à 2 atomes de carbone et 1 à 5 atomes d'halogène identiques ou différents, de méme qu'un phényle éventuellement substitué par de l'halogène,

$R^2$ représente un alcoyle à chaîne droite ou ramifiée ayant 1 à 4 atomes de carbone, en outre un t-butyle substitué par de l'halogène ou un aryle ayant 6 à 10 atomes de carbone eventuellement substitué une ou plusieurs fois, de manière identique ou différente, en citant comme substituants, de l'halogène, alcoyle ayant 1 à 4 atomes de carbone, alcoxy ayant 1 à 4 atomes de carbone, nitro, halogénoalcoyle ayant 1 à 2 atomes de carbone et 1 à 5 atomes d'halogène identiques ou différents, de même qu'un phényle éventuellement substitue par de l'halogene,

X' représente du brome ou de l'iode, de même aussi que de l'hydrogène lorsque non en même temps, $R^1$ représente un aryloxy éventuellement substitué, $R^2$ un alcoyle ou un aryle éventuellement substitué et A un atome d'azote,

A représente de l'azote ou le groupe CH et

n les nombres 0 ou 1,

de même que leurs sels d'addition d'acides et leurs complexes avec des sels métalliques.

3. Dérivés alcinyl-azoliques de formule générale (Ia) selon la revendication 2, dans laquelle

$R^1$ représente un phényle ou phénoxy éventuellement substitués une à trois fois, de manière identique ou différente, en citant comme substituants: fluor, chlore, brome, méthyle, méthoxy, nitro, trifluorométhyle, phényle et chlorophényle,

$R^2$ represente un t-butyle substitue une ou deux fois par du fluor, du chlore ou du brome ou un phenyle eventuellement substitué une à trois fois, de manière identique ou differente, en envisageant comme substituants les phényl-substituants déjà cités pour $R^1$,

X' représente du brome et de l'iode, de même aussi que de l'hydrogène lorsque, non en même temps, $R^1$ représente un phenoxy éventuellement substitué, $R^2$ un t-butyle ou un phényle éventuellement substitué et A un atome d'azote , et

A et l'indice n ont la signification indiquee à la revendication 2.

4. Procédé de fabrication de dérivés alcinyl-azoliques de formule générale (Ia)

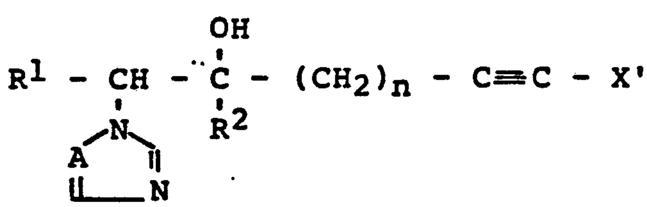 (Ia)

dans laquelle

$R^1$ représente un aryle ou aryloxy ayant chacun 6 à 10 atomes de carbone et éventuellement substitués une ou plusieurs fois, de manière identique ou différente, en citant comme substituants halogène, alcoyle ayant 1 à

4 atomes de carbone, alcoxy ayant 1 à 4 atomes de carbone, nitro, halogénoalcoyle ayant 1 à 2 atomes de carbone et 1 à 5 atomes d'halogène identiques ou différents, de même qu'un phényle éventuellement substitué par de l'halogène,

$R^2$ représente un alcoyle à chaine droite ou ramifiée ayant 1 à 4 atomes de carbone, en outre un t-butyle substitué par de l'halogène ou un aryle ayant 6 à 10 atomes de carbone éventuellement substitué une ou plusieurs fois, de manière identique ou différente, en citant comme substituants halogène, alcoyle ayant 1 à 4 atomes de carbone, alcoxy ayant 1 à 4 atomes de carbone, nitro, halogénoalcoyle ayant 1 à 2 atomes de carbone et 1 à 5 atomes d'halogène identiques ou différents, de même qu'un phényle éventuellement substitué par de l'halogène,

X' représente du brome ou de l'iode, de même aussi que de l'hydrogène lorsque, non en même temps, $R^1$ représente un aryloxy éventuellement substitué, $R^2$ un alcoyle ou un aryle éventuellement substitué et A un atome d'azote,

A représente de l'azote ou le groupe CH et

n représente les nombres 0 ou 1,

et de leurs sels d'addition d'acides et de leurs complexes avec des sels métalliques, caractérisé en ce qu'on fait réagir des azolyl-cétones de formule (II)

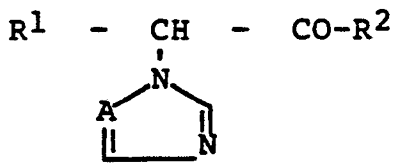

(II)

dans laquelle

A, $R^1$ et $R^2$ ont la signification indiquée plus haut,

a) dans le cas de n = 0,

avec des mono-sels de métaux alcalins ou des monocomposés de Grignard de l'acétylène en présence d'un solvant organique aprotique, comme par exemple du tétrahydrofuranne, à des températures entre 0 et 50°C et on hydrolyse ensuite le mélange de réaction , ou bien

b) dans le cas de n = 1,

avec des halogenures de propargyle en présence d'aluminium activé et en présence d'un solvant organique aprotique, comme par exemple du tétrahydrofuranne, à des températures entre -70 et +30°C et en ce qu'éventuellement on fait réagir les composés obtenus par les procédés (a) et (b), de formule (Id) :

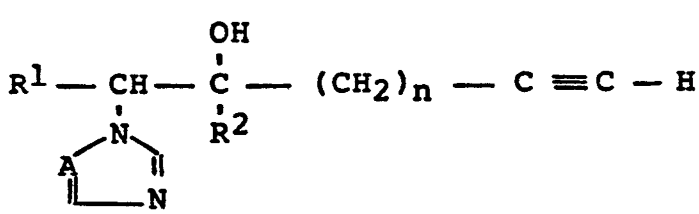

(Id)

dans laquelle

A, $R^1$, $R^2$ et l'indice n ont la signification indiquée plus haut, de la manière usuelle avec du brome ou de l'iode en présence d'un diluant, à des températures entre 0 et 50°C, et en ce qu'éventuellement on fixe encore sur les composés de formule (Ia) ainsi obtenus un acide ou un sel métallique.